# FASCICULE DE BREVET EUROPEEN

(11) **EP 2 656 065 B1**
(45) Date de publication et mention de la délivrance du brevet: **20.04.2016**
(21) Numéro de dépôt: 11813359.4
(22) Date de dépôt: 20.12.2011
(51) Int. Cl.: G01N 33/18, G01F 1/075, G01N 27/28

(54) **DISPOSITIF DE CONTRÔLE DU POUVOIR OXYDANT DANS DE L'EAU EN CIRCULATION, S'APPLIQUANT NOTAMMENT À L'EAU D'UNE PISCINE**
VORRICHTUNG ZUR ÜBERPRÜFUNG DER OXIDATIONSLEISTUNG VON UMLAUFWASSER, BESONDERS FÜR DAS WASSER EINES SCHWIMMBECKENS
DEVICE FOR CHECKING THE OXIDISING POWER OF CIRCULATING WATER, IN PARTICULAR USEFUL FOR THE WATER OF A SWIMMING POOL

(30) Priorité: 20.12.2010 FR 1004971
(43) Date de publication de la demande: 30.10.2013
(73) Titulaire: Ducoeur, Dominique, 95520 Osny (FR)
(72) Inventeur: Ducoeur, Dominique, 95520 Osny (FR)
(74) Mandataire: Thibon, Norbert
(86) Numéro de dépôt international: PCT/IB2011/003097
(87) Numéro de publication internationale: WO 2012/085641

(56) Documents cités:
- CN-A- 101 660 408
- DE-A1- 2 037 497
- FR-A1- 2 715 222
- GB-A- 2 242 022
- US-A- 3 959 087
- US-A- 4 033 830
- US-A- 6 079 280

## Description

La présente invention concerne la mesure du pouvoir oxydant dans de l'eau en circulation, en visant notamment, en application particulière le contrôle du pouvoir dans l'eau d'une piscine et le réglage du taux de chlore dans l'eau d'une telle piscine en fonction du pouvoir oxydant de l'eau détecté dans une canalisation de recirculation d'eau en circuit fermé avec la piscine.

Ce dispositif permet de faire intervenir une mesure de débit sur un courant d'eau en circulation pour ajuster une mesure ampérométrique du pouvoir oxydant de l'eau en circulation. Le pouvoir oxydant mesuré par ampérométrie est corrigé en fonction du résultat de la mesure de débit. Suivant l'invention, des moyens capteurs d'ampérométrie et des moyens capteurs de débit, procédant avantageusement par voie électromagnétique, sont incorporés dans un même boîtier de sonde se montant latéralement dans une canalisation de circulation de l'eau à analyser, à travers la paroi de ladite canalisation, de manière à immerger l'ensemble des moyens capteurs dans l'eau en circulation dans cette canalisation.

Le dispositif suivant l'invention se distingue ainsi de l'art antérieur illustré par exemple par le brevet américain, US 4 033 830, dans le lequel les différents éléments constitutifs des moyens capteurs sont montés séparément de part et d'autre de la conduite d'eau. Il s'en distingue aussi par tous les avantages qui découlent d'une construction compacte en usine, y compris du point de vue de la disposition relative des éléments qui le constituent, de la précision des réglages, de la reproductibilité des conditions de fonctionnement et des résultats des mesures, de la commodité de manipulation de l'ensemble et de la sécurité lors du montage du dispositif sur une canalisation d'installation existante. Il s'y ajoute que contrairement à ce que l'on connaissait d'autres dispositifs de l'art antérieur, par exemple celui décrit dans le brevet américain US 359 087, l'invention permet d'assurer en continu le contrôle de la teneur en chlore de l'eau d'une piscine directement dans la canalisation de recirculation d'eau en circuit fermé dans laquelle s'opère l'ajout de chlore (ou autre composé à propriétés oxydantes). En effet, la mesure de débit effectuée concommittament à la mesure ampérométrique de pouvoir oxydant (mesure du courant électrique résultant des variations de la différence de potentiel entre deux électrodes plongées dans l'eau à analyser) permet d'assurer en continu un ajustement de la concentration d'oxydant mesurée en fonction de l'information de débit obtenue au même endroit dans l'eau en circulation, et il n'est alors plus besoin de prévoir une dérivation à débit constant pour la mesure amérométrique.

Un tel dispositif est décrit dans le brevet allemand DE 2 037 497. Selon des caractéristiques de l'invention dans ses modes de mise en oeuvre préférés, les moyens capteurs de débit ("débitmètre" ci-après) et les moyens capteurs ampérométriques ("ampéromètre" ci-après) sont assemblés mécaniquement les uns autres à l'intérieur du boîtier de sonde. Ils se combinent dans un même assemblage mécanique, les moyens de l'un servant de support aux moyens de l'autre.

C'est ainsi notamment que l'on prévoit avantageusement de constituer le débitmètre par une turbine montée librement rotative sous l'effet du flux d'eau traversant le boîtier et que dans ce cas, l'axe fixe de rotation de la turbine est aussi l'une des électrodes de l'ampéromètre. C'est ainsi aussi qu'une électrode plate de l'ampéromètre se plaçant dans le fil de l'eau en circulation peut avantageusement servir de référentiel dans des moyens compte-tours électromagnétiques fournissant une information caractéristique de la vitesse de rotation de la turbine par coopération avec un aimant disposé à l'extrémité de la turbine qui est en regard de l'électrode de référence.

Selon d'autres caractéristiques de l'invention dans ses modes de mise en oeuvre préférés, le boîtier de sonde comporte des fenêtres pour l'entrée et la sortie de l'eau qui s'étendent respectivement en regard d'un débitmètre choisi du type électromagnétique à turbine d'une part et de l'ampéromètre d'autre part. De préférence, l'entrée d'eau au niveau de l'électrode plate de l'ampéromètre s'effectue par une fenêtre allongée radialement parallèlement à ladite électrode plate, alors que sur la hauteur de la turbine, parallèlement à son axe, entrée et sortie d'eau s'effectuent par des fenêtres allongées axialement. La fenêtre radiale correspondant à l'ampéromètre assure un flux laminaire le long de l'électrode plate. Elle peut avantageusement comporter un moyen de réglage de son ouverture, ce qui permet d'adapter le dispositif au débit nominal prévu dans l'installation à laquelle il est destiné.

Le dispositif intègre des moyens de nettoyage des moyens de l'ampéromètre automatiquement associés au fonctionnement du débitmètre. Ainsi, le dispositif est auto-nettoyé, le mouvement de la turbine débitmétrique assurant le nettoyage des électrodes de l'ampéromètre. Ces moyens de nettoyage comportent notamment une brosse montée à l'extrémité de la turbine en regard de l'électrode plate de l'ampéromètre, de sorte que la rotation de la turbine entraîne le brossage de ladite électrode plate. D'autre part, un faible jeu de montage de l'axe de rotation de la turbine autour de la contre-électrode de l'ampéromètre peut permettre d'assurer par la rotation de la turbine le décrassage de la contre-électrode.

L'invention sera maintenant plus complètement décrite dans le cadre de caractéristiques préférées et de leurs avantages, en faisant référence aux figures dans lesquelles :
- la figure 1 représente une vue d'ensemble d'une canalisation de recirculation d'eau d'une piscine dans laquelle on intègre un dispositif de mesure selon l'invention ;
- la figure 2 est une vue en élévation d'une partie de la canalisation illustrée sur la figure 1 et d'un dispositif de mesure selon l'invention qui est associé à la canalisation ;
- la figure 3 est une vue en élévation du dispositif de mesure illustré sur la figure 2 ;
- la figure 4 est une vue éclatée du dispositif de mesure illustré sur la figure 3 ;
- la figure 5 est une vue en coupe du dispositif de mesure illustré sur la figure 4, avec le carter déposé ;
- et la figure 6 est une vue de détail d'un dispositif de mesure selon un deuxième mode de réalisation de l'invention.

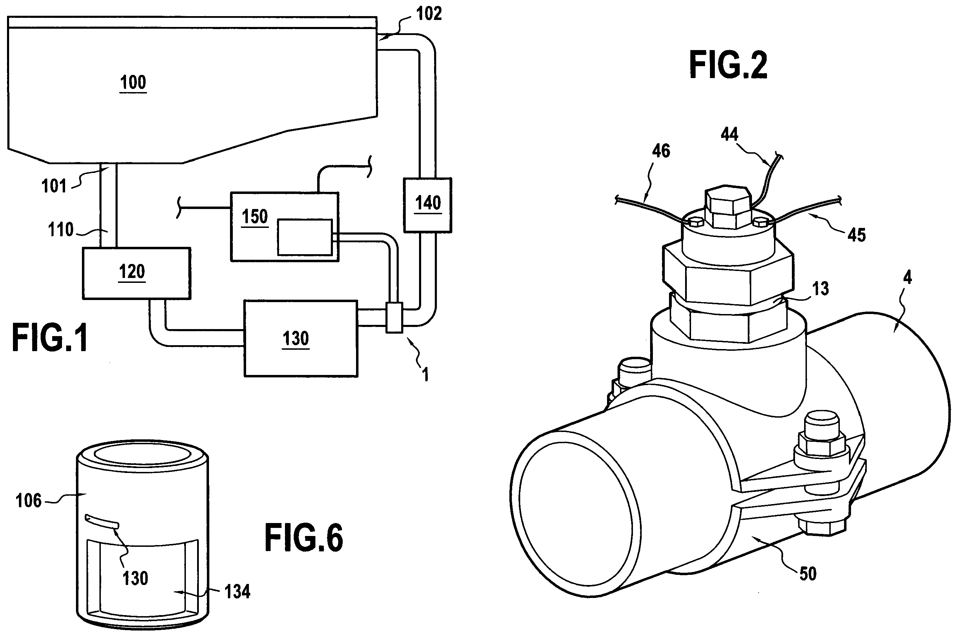

La figure 1 illustre une piscine équipée conformément à la présente invention d'un dispositif de contrôle du pouvoir oxydant de l'eau de la piscine dont les moyens capteurs sont intégrés avec des moyens capteurs de débit dans une sonde 1 qui est montée sur la conduite principale de recirculation d'eau pour addition de chlore, sans qu'une dérivation spécifique soit nécessaire. Le circuit de recirculation d'eau 110, ici celui d'une piscine privée 100, s'étend depuis une bonde d'évacuation d'eau 101 vers un piquage de retour d'eau 102, en passant successivement par un poste de filtration 120, un poste de chauffage 130, un poste de traitement chimique de l'eau 140, en formant un circuit fermé avec la masse d'eau présente dans la piscine.

Etant entendu que la sonde du dispositif suivant l'invention pourra être montée à n'importe quel endroit du circuit sans sortir du contexte de l'invention et que les postes ici représentés pourraient être différents ou différemment agencés pour un autre circuit de recirculation d'eau d'une autre piscine, on observe que, dans l'exemple de réalisation représenté, la sonde est montée dans le circuit en aval du poste de chauffage et en amont du poste de traitement chimique. La sonde est reliée électriquement à des moyens d'ajustement paramétrés pour calculer automatiquement le pouvoir oxydant de l'eau en corrigeant le résultat d'une mesure ampérométrique, fourni par des moyens capteurs sensibles à la différence de potentiel entre deux électrodes immergées dans l'eau à analyser, en fonction du débit de cette eau, tel que mesuré au même endroit par des moyens capteurs de débit procédant avantageusement par voie électronmagnétique.

On va décrire maintenant plus précisément la sonde, telle qu'elle est notamment illustrée sur les figures 2 à 5. Le dispositif suivant l'invention comporte un boîtier de sonde 2 adapté à être monté en piquage latéral dans la canalisation, par une ouverture 3 réalisée à travers la paroi du tube 4 de la canalisation. Le boîtier comporte une partie immergée de mesure 6, qui se loge alors à l'intérieur du tube et qui contient les différents moyens capteurs, et une partie de raccordement 8 portant les raccords électriques qui conduisent l'information captée aux moyens de calcul extérieurs.

La partie immergée comporte un carter 10 protégeant des moyens capteurs qui sont confinés au voisinage les uns des autres à l'intérieur de cette partie immergée du boîtier.

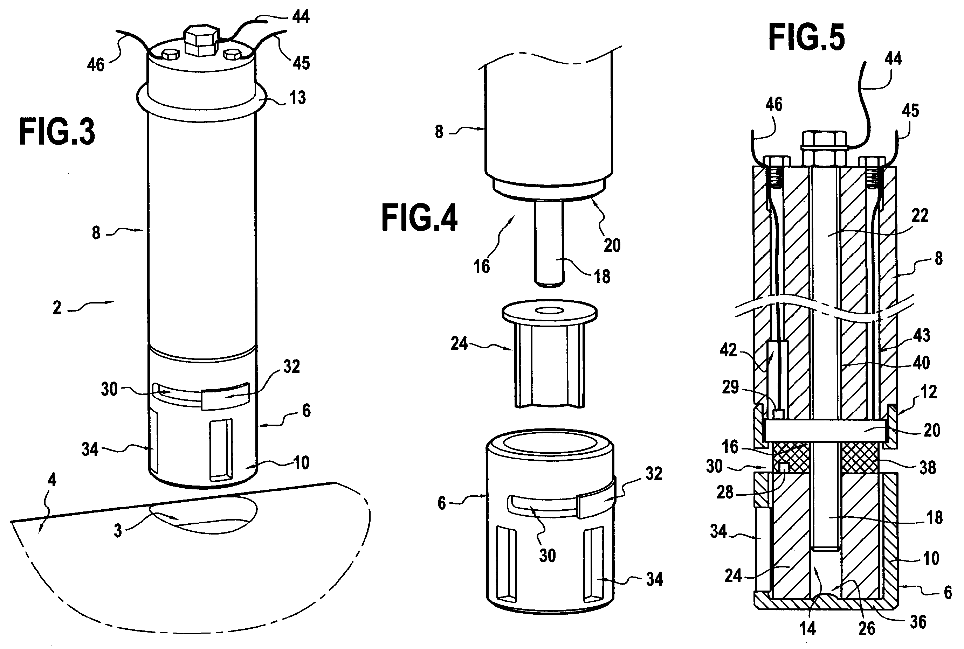

Le carter comporte des fenêtres de passage pour la circulation d'eau à travers la partie immergée du boîtier. Le carter comporte en outre des moyens de fixation 12 sur la partie de raccordement du boîtier pour que la partie immergée et la partie de raccordement forment ensemble le boîtier (visible sur la figure 5).

La partie de raccordement du boîtier loge des éléments de connexion électrique pour se raccorder avec des moyens de calcul du pouvoir oxydant de la masse d'eau, et notamment des fils reliant les moyens capteurs présents à l'intérieur du boîtier à ces moyens de calcul disposés à l'extérieur du boîtier, hors de la canalisation. Les fils débouchent à l'extérieur du boîtier pour être raccordés auxdits moyens de calcul.

Une gorge est réalisée sur le pourtour de la paroi extérieure de la partie de raccordement du boîtier, pour y loger un joint annulaire d'étanchéité 13, qui dépasse en saillie de la paroi extérieure, à la jonction entre les deux parties.

Les moyens capteurs confinés dans le boîtier comportent un débitmètre électromagnétique 14 et un ampéromètre 16.

L'ampéromètre comporte dans l'exemple illustré deux électrodes 18 et 20. On mesure l'intensité du courant entre les électrodes qui est sensible à la différence de potentiel entre le potentiel fixe d'une électrode de référence 18 et celui d'une électrode de mesure 20 dont le potentiel varie en fonction du taux d'oxydant dans le volume compris à un instant donné entre les deux électrodes.

Avantageusement, l'électrode de mesure est plate et l'électrode de référence est formée par une contre-électrode qui s'étend en saillie du centre de l'électrode plate. L'électrode de mesure s'étend transversalement à l'axe du boîtier et parallèlement au plan du flux d'eau en circulation dans le tube. Cette électrode de mesure s'étend entre la partie immergée et la partie de raccordement et elle est rendue solidaire de la partie de raccordement. La contre-électrode est prolongée de l'autre côté de l'électrode plate par une tige de fixation 22 dans la partie de raccordement.

Comme décrit précédemment, le débitmètre est placé au voisinage de l'ampéromètre dans la partie immergée du boîtier, et il est avantageusement porté par l'ampéromètre. Dans le mode de réalisation illustré, il comporte une turbine 24 adaptée à se déplacer en rotation sous l'effet du courant de l'eau en circulation dans le tube et un moyen compte-tours pour déterminer la vitesse de rotation de la turbine par rapport à une référence.

La turbine est montée libre en rotation autour de la contre-électrode. Le carter de la partie immergée comporte en son fond une butée de maintien 26 en position axiale de la turbine. La position verticale selon laquelle le dispositif est disposé dans le mode de réalisation décrit permet par gravité d'appliquer la turbine contre cette butée. Dans cette position en butée, un espace est laissé entre les faces en regard de l'électrode plate et de la turbine pour le passage de l'eau le long de l'électrode plate et pour permettre la rotation de la turbine.

Le moyen compte-tours comporte dans l'exemple de réalisation un aimant 28 disposé sur la face de la turbine en regard de l'électrode plate et un capteur magnétique 29 fixe pour détecter les passages de l'aimant. Le capteur est disposé dans la partie de raccordement de sorte que l'électrode plate s'étend entre l'aimant et le capteur, le capteur étant dimensionné pour détecter chaque passage de l'aimant à travers l'électrode plate.

Les fenêtres aménagées dans le boîtier dans sa partie immergée permettent le passage de l'eau à travers le boîtier et donc au contact des capteurs sans perturber la circulation de l'eau.Ces fenêtres sont respectivement associées à chacun des moyens capteurs pour que des flux de liquide restent propres à chaque moyen capteur et qu'ainsi l'action du débitmètre sur l'eau ne perturbe pas le flux qu'on souhaite laminaire pour l'ampéromètre.

Une fenêtre radiale 30 est ainsi associée à l'ampéromètre, ladite fenêtre s'étendant suivant un axe parallèle au plan dans lequel s'étend l'électrode plate de mesure.

Des moyens de réglage de l'ouverture 32 de cette fenêtre radiale sont prévus pour que l'utilisateur puisse fixer une section de passage spécifique. La valeur de cette section est connue des moyens de calcul vers lesquels sont envoyées les données mesurées par l'ampéromètre et le débitmètre. Ainsi, les moyens de calcul répertorient, comme données en provenance de l'ampéromètre, une section donnée et une valeur donnée d'oxydant dans l'eau. Comme cela sera décrit ci-après et comme visible sur la figure 6, on peut prévoir une fente radiale de largeur axiale faible pour limiter le flux d'eau passant à la surface de.

Deux fenêtres axiales 34 sont en outre associées au débitmètre. Elles s'étendent le long du carter en regard de la turbine, sur toute la hauteur de la turbine entre la fenêtre radiale et le fond du carter 36. Les deux fenêtres sont décalées angulairement sur le pourtour du carter, avec une fenêtre axiale orientée vers l'amont de la circulation d'eau tandis que l'autre fenêtre est orientée vers l'aval du sens de circulation. La fenêtre axiale amont permet l'entrée d'eau dans le boîtier au contact de la turbine et la fenêtre axiale avale permet la sortie d'eau en formant à l'intérieur du carter un conduit qui guide l'eau sur la turbine. La section de ce conduit sert de base au calcul du débit d'eau en fonction de la vitesse de rotation de la turbine sous l'effet du courant d'eau.

Comme illustré sur les figures pour une conduite d'eau horizontale, la disposition est ici essentiellement verticale, avec un boîtier qui est rapporté dans le tube par le dessus et donc une partie immergée qui s'étend sous la partie de raccordement. Le débitmètre est sous l'ampéromètre de sorte que les fenêtres de passage, qui reproduisent le même agencement que celui des moyens de mesure, sont disposées avec les fenêtres axiales sous la fenêtre radiale.

Des moyens de nettoyage de l'ampéromètre sont prévus pour être automatiquement associés au fonctionnement du débitmètre. D'une part, une brosse 38 est montée sur la face d'extrémité de la turbine en regard de l'électrode plate de l'ampéromètre et d'autre part, la turbine est montée autour de la contre-électrode avec un jeu de montage. De la sorte, la rotation de la turbine assure d'une part le nettoyage par brossage de l'électrode plate et d'autre part le décrassage de la contre-électrode par débattement de la turbine contre la contre-électrode lui servant d'axe de rotation. C'est par la rotation de la turbine qu'on s'assure du nettoyage automatique à la fois de la contre-électrode et de l'électrode plate de sorte que le nettoyage des électrodes est automatique et assuré dans le temps.

La partie de raccordement du boîtier de sonde comporte un alésage axial central 40 et deux alésages axiaux périphériques 42 et 43 qui s'étendent sur toute la hauteur de la partie de raccordement depuis l'électrode plate, qui s'étend transversalement à une extrémité de cette partie de raccordement, jusqu'à l'extrémité opposée. A cette extrémité, les alésages sont bouchés par des vis qui autorisent le passage vers l'extérieur des fils de connexion reliés à des composants à l'intérieur du boîtier.

L'alésage central loge la tige de fixation de la contre-électrode. Le long de la tige, un premier fil de connexion 44 s'étend vers l'extérieur du boîtier pour transmettre des données aux moyens de calcul relativent au potentiel mesuré de l'électrode plate de mesure qui réagit lors du passage de l'eau. Ce potentiel est comparé avec le potentiel de référence de la contre-électrode, pour donner une mesure ampérométrique représentative du pouvoir oxydant de l'eau en circulation.

Un premier alésage axial périphérique 42 s'étend parallèlement à l'alésage central et le capteur magnétique, composante du compte-tours, est logé dans ce premier alésage périphérique pour compter les impulsions magnétiques à chaque passage de la turbine et de l'aimant rapporté sur cette turbine. Le capteur magnétique envoie les informations relatives à ce décompte des impulsions vers les moyens de calcul par l'intermédiaire d'un deuxième fil de connexion 46 qui remonte l'alésage axial périphérique.

Un deuxième alésage axial périphérique 43 s'étend parallèlement à l'alésage central pour loger un troisième fil de connexion 45 qui communique vers les moyens de calcul le potentiel de l'électrode plate de référence.

Les fils de connexion s'étendent alors hors du tube vers des moyens de calcul adaptés à déterminer un pouvoir oxydant d'une masse d'eau sur la base des informations qui lui sont transmises relatives à la circulation d'une eau en dérivation de cette masse d'eau.

Comme illustré sur les figures et plus particulièrement sur les figures 2 et 3, le dispositif est monté directement dans la canalisation.

On réalise à cet effet un alésage à travers la paroi tubulaire dans le tube de la canalisation pour former l'ouverture latérale 3, avantageusement sur le dessus comme cela a été décrit précédemment afin de faciliter les questions d'étanchéité du dispositif.

On insère le boîtier dans le tube de la canalisation jusqu'à ce que le joint annulaire d'étanchéité 13 entre en butée avec les bords délimitant l'ouverture latérale. Dans cette position, la partie immergée se trouve complètement à l'intérieur du tube et la partie de raccordement s'étend en partie à l'extérieur du tube, de sorte que les fils électriques de raccordement ne pénètrent pas dans le tube. On raccorde ces fils aux moyens de calcul, à l'abri de l'eau en circulation.

Une bride de serrage 50 est montée autour de la canalisation pour maintenir en position le boîtier par rapport à la canalisation. Cette bride peut déjà être en place autour du tube avant l'insertion du boîtier et être resserrée par la suite pour réaliser le maintien en position de l'ensemble.

La construction du dispositif et son placement transversal à la canalisation assurent une disposition de l'électrode plate dans la direction de la circulation d'eau pour qu'elle soit constamment léchée par le courant d'eau.

On va maintenant décrire le fonctionnement du dispositif de contrôle de taux d'oxydant selon l'invention.

Lorsque le boîtier est en place dans le tube de la canalisation, l'eau en circulation dans cette traverse le boîtier placé en travers de son chemin. L'eau passe simultanément dans la fenêtre radiale et dans la fenêtre axiale d'entrée d'eau, et alimente simultanément la turbine et les électrodes. On peut prévoir de prolonger vers l'intérieur du boîtier les surfaces délimitant les bords des fenêtres pour guider plus distinctement l'eau vers l'un ou l'autre des moyens de mesure.

Le taux d'oxydant, et par exemple de chlore, de l'eau en circulation est alors mesuré au niveau des électrodes selon le principe de mesure décrit précédemment et la valeur mesurée est transmise aux moyens de calculs par l'intermédiaire des fils de connexion électriques.

Simultanément, une valeur représentative du débit de cette eau en circulation est mesurée en prenant en compte la vitesse de l'eau passant dans le boîtier. Le courant entraîne en rotation la turbine montée libre en rotation autour de la contre-électrode. Le compte-tours permet de déterminer la vitesse de rotation de la turbine. La valeur mesurée par le compte tours est transmise aux moyens de calculs par l'intermédiaire des fils de connexion électriques.,

Les moyens de calcul collectent les données mesurées, à savoir le taux de chlore, ou de brome par exemple, de l'eau en circulation dans la canalisation et la vitesse de rotation de la turbine générée par le passage de cette eau dans cette même canalisation. Ces données s'ajoutent aux valeurs déjà présentes dans la base de données des moyens de calculs dans laquelle on retrouve la valeur de section de passage des fenêtres axiales et radiales. Un premier calcul est effectué pour connaître le débit de l'eau dans ce passage en fonction de la section et de la valeur de vitesse mesurée. On fait alors un ajustement de la valeur mesurée du taux de chlore au niveau de l'ampéromètre en fonction du débit en utilisant des cartographies pré-enregistrées, permettant de donner un pouvoir oxydant dans la masse d'eau de la piscine sur la base d'un taux d'oxydant d'une eau en recirculation et de la vitesse de débit de cette eau en recirculation.

Ainsi, on peut selon l'invention utiliser la valeur du débit de l'eau calculé au niveau du débitmètre pour donner une valeur réaliste du débit au niveau de l'ampéromètre, du fait de la position proche des moyens capteurs dans la canalisation, et avantageusement dans le même boîtier. On observe qu'il est intéressant de réaliser un étalonnage précis pour obtenir une table des valeurs fiable sachant mettre en relation un pouvoir d'oxydant et un débit d'eau en circulation avec une valeur de pouvoir oxydant présent dans la masse d'eau associée à cette eau en circulation.

Une fois le calcul du taux effectif d'oxydant dans l'eau effectué, cette information est envoyée en temps réel vers les différents postes pouvant être impactés par cette information, notamment vers le poste de traitement chimique pour permettre un dosage d'oxydant adapté à la valeur mesurée.

La description qui précède explique clairement comment l'invention permet d'atteindre les objectifs qu'elle s'est fixés et notamment d'assurer un contrôle du pouvoir oxydant d'une masse d'eau par des mesures à même les conduits de circulation d'eau principaux, en tenant compte des éventuelles variations de débit inhérentes à ces conduites principales de recirculation d'eau. Ainsi, si un filtre à sable par exemple qui s'étend en travers de la circulation d'eau est encrassé, il fait varier le débit d'eau et il fait donc varier le nombre de particules d'oxydant détectables par ampérométrie à un instant donné. La mesure simultanée du débit d'eau permet de comprendre que la valeur plus faible du pouvoir oxydant n'est pas due à un faible pouvoir oxydant de la masse d'eau associée mais qu'elle est due à des variations de débit dans la canalisation où sont faites les mesures. Il n'est alors peut-être pas nécessaire de délivrer du chlore dans la piscine et on peut éviter ainsi un surdosage en chlore ou tout autre oxydant dans la piscine. L'ajustement nécessaire de la mesure du taux d'oxydant selon le débit de l'eau peut en outre être utilisé pour lancer une alerte quant à la nécessité de désencrasser le filtre lorsque le débit mesuré passe en dessous d'un certain seuil. On peut ainsi avantageusement se passer de la présence d'un capteur de pression, classiquement utilisé en aval du filtre pour contrôler son encrassement, puisque l'analyse du débit d'eau passé à travers le filtre et de son taux d'oxydant permet de tirer un enseignement sur la faculté du filtre à laisser passer l'eau. Le temps que le filtre soit nettoyé ou changé, le contrôle par le dispositif du pouvoir oxydant de la masse d'eau reste fiable car on continue à s'adapter en fonction de la mesure du nombre de tours par minute de la turbine.

On va décrire maintenant un deuxième mode de réalisation de l'invention, et plus particulièrement une partie immergée 106 du boîtier différente de la partie immergée 6 décrite dans le premier mode de réalisation. Cette partie immergée 106 est formée d'un carter dans lequel la fenêtre de passage radial de l'eau vers l'ampéromètre est réalisée par une fente 130 de dimension fixe. On peut observer que la fente 130 est plus étroite que la fenêtre radiale 30 du premier mode de réalisation. On limite ainsi l'apport d'eau sur les électrodes pour optimiser la détection d'oxydant. La partie immergée 106 présente en outre une fenêtre unique de passage d'eau vers la turbine 134. On oriente le boîtier dans la canalisation d'eau de sorte que cette fenêtre 134 reçoive l'eau tangentiellement au carter. L'eau impacte ainsi l'extrémité des pâles de la turbine et la rotation de cette turbine est améliorée.

On peut prévoir de rapporter, sur une partie de raccordement standard, une partie immergée 106 de forme équivalente mais plus longue, pour adapter le dispositif à des tubes de circulation d'eau de plus grand diamètre. On propose alors des têtes interchangeables dans lesquelles la taille de la fente peut être agrandie en proportion à l'agrandissement du carter puisque l'utilisation du dispositif dans un tube plus large implique un volume d'eau en circulation plus important. On peut prévoir également dans ce cas d'associer au carter plus long une hélice plus longue.

D'autres variantes non représentées sont prévues et certaines sont évoquées ci-dessous, sans toutefois que cette liste soit exhaustive :
- On prend en compte les variations éventuelles de la conductivité de l'eau en fonction de la température ; à cet effet, on intègre dans le boîtier un capteur de température que l'on dispose à proximité du débitmètre ;
- On prévoit de se passer des fils de connexion entre les moyens de mesure et les moyens de calcul et de faire passer les informations de l'un à l'autre par radiofréquence, avec des émetteurs associés au débitmètre et à l'ampéromètre et au moins un récepteur associé aux moyens de calcul ;
- La forme de l'hélice est différente de l'hélice 24 illustrée sur la figure 4 et dans laquelle les pales sont droites ; on prévoit ainsi des pales de forme courbe ; il convient alors de placer la turbine de sorte que l'eau en circulation passant par les fenêtres 34 ou 134 arrive sur une surface concave des pales.

Il ressort néanmoins de ce qui précède que l'invention n'est pas limitée aux modes de mise en oeuvre qui ont été spécifiquement décrits et représentés sur les figures.

## Revendications

1. Dispositif de contrôle du pouvoir oxydant de l'eau en circulation dans une canalisation comportant des moyens capteurs de débit (14) et des moyens capteurs par ampérométrie du taux d'oxydant dans l'eau (16), qui sont assemblés les uns aux autres à l'intérieur d'un même boîtier de sonde (2) se montant en piquage latéral à travers ladite canalisation de sorte que lesdits moyens capteurs soient immergés dans l'eau en circulation, **caractérisé en ce que** lesdits moyens capteurs de débit consistent en un débitmètre (14) comportant une turbine tandis que lesdits moyens capteurs du taux d'oxydant consistent en un ampéromètre (16) comportant deux électrodes (18, 20) dont l'une s'étend dans l'axe dudit boîtier et porte ladite turbine du débitmètre (14), montée libre en rotation autour d'elle.

2. Dispositif selon la revendication 1, **caractérisé en ce que** ledit boîtier (2) est à carter de section circulaire.

3. Dispositif selon la revendication 1 ou 2, **caractérisé en ce qu'**il comporte en outre des moyens de maintien en position axiale de la turbine.

4. Dispositif selon la revendication 3, **caractérisé en ce que** les moyens de maintien en position consistent en une butée (26) disposée au fond du boîtier de sonde (2).

5. Dispositif selon l'une quelconque des revendications précédentes, **caractérisé en ce que** l'ampéromètre (16) comporte une électrode plate (20) et une contre-électrode (18) en saillie de ladite électrode, ladite contre-électrode étant celle des deux électrodes qui porte le débitmètre (14).

6. Dispositif selon la revendication 5, **caractérisé en ce que** le débitmètre (14) dont la turbine (24) montée libre en rotation autour de la contre-électrode (18) de l'ampéromètre comporte des moyens compte-tours (28, 29).

7. Dispositif selon l'une quelconque des revendications 1 à 6, **caractérisé en ce que** ledit boîtier (2) comporte des fenêtres (30, 34) pour l'entrée et la sortie de l'eau qui s'étendent respectivement en regard du débitmètre (14) et de l'ampéromètre (16).

8. Dispositif selon la revendication 7, **caractérisé en ce qu'**une fenêtre (30) est aménagée radialement dans la surface du carter (10) en regard de l'ampéromètre (16).

9. Dispositif selon la revendication 8, **caractérisé en ce que** ladite fenêtre (30) comporte un moyen de réglage (32) de l'ouverture de ladite fenêtre.

10. Dispositif selon l'une des revendications 7 à 9, caractérisé en que des fenêtres d'entrée et de sortie d'eau (34) sont aménagées axialement dans la surface du carter, en regard du débitmètre (14).

11. Dispositif selon l'une des revendications 3 à 10, **caractérisé en ce qu'**il intègre des moyens de nettoyage de l'ampéromètre (16) automatiquement associés au fonctionnement du débitmètre (14).

12. Dispositif selon la revendication 11, **caractérisé en ce que** les moyens de nettoyage comportent une brosse (38) adaptée à frotter l'ampéromètre (16) par l'action du débitmètre (14).

13. Piscine comportant un dispositif de contrôle selon l'une des revendications 1 à 12, **caractérisée en ce que** le dispositif de contrôle se monte dans une canalisation de recirculation d'eau, qui forme un circuit fermé avec la masse d'eau de la piscine.

14. Piscine selon la revendication 13, **caractérisée en ce que** le dispositif de mesure se monte dans ladite canalisation de sorte que l'électrode plate du dispositif s'étende dans le plan de circulation d'eau.

15. Piscine selon l'une des revendications 13 ou 14, **caractérisée en ce qu'**un filtre est disposé dans ladite canalisation de recirculation d'eau, en amont du dispositif de contrôle, l'encrassement du filtre étant détecté par les valeurs de débit de l'eau en circulation dans la canalisation mesurées par le dispositif de contrôle du taux d'oxydant.

## Patentansprüche

1. Vorrichtung zur Überprüfung des Oxidationsvermögens von in einer Kanalisation zirkulierendem Wasser, wobei die Vorrichtung umfasst
Durchflusssensormittel (14) und amperometrische Sensormittel zum Ermitteln des Gehalts an Oxidationsmittel im Wasser (16), die miteinander in einem gleichen Sondengehäuse (2) angeordnet sind, indem sie sich seitlich verzweigend über die Kanalisation hinweg angebracht sind, derart, dass die Sensormittel in das zirkulierende Wasser eingetaucht sind, **dadurch gekennzeichnet, dass**
die Durchflusssensormittel aus einem Durchflussmesser (14) bestehen, der ein Laufrad aufweist, wohingegen die Sensormittel zum Ermitteln des Gehalts an Oxidationsmittel aus einem Amperemeter (16) bestehen, das zwei Elektroden (18, 20) aufweist, von denen sich eine entlang der Achse des Gehäuses erstreckt und das Laufrad des Durchflussmessers (14) trägt, das drehbeweglich um sie befestigt ist.

2. Vorrichtung nach Anspruch 1, **dadurch gekennzeichnet, dass** das Gehäuse (2) einen Schutzmantel mit kreisförmigem Querschnitt aufweist.

3. Vorrichtung nach Anspruch 1 oder 2, **dadurch gekennzeichnet, dass** sie weiterhin Haltemittel in axialer Position des Laufrades aufweist.

4. Vorrichtung nach Anspruch 3, **dadurch gekennzeichnet, dass** die Positionshaltemittel aus einem Anschlag (26) am Boden des Sondengehäuses (2) bestehen.

5. Vorrichtung nach einem der vorangehenden Ansprüche, **dadurch gekennzeichnet, dass** das Amperemeter (16) eine Plattenelektrode (20) und eine von der Elektrode emporragende Gegenelektrode (18) umfasst, wobei die Gegenelektrode diejenige der beiden Elektrode ist, die den Durchflussmesser (14) trägt.

6. Vorrichtung nach Anspruch 5, **dadurch gekennzeichnet, dass** der Durchflussmesser (14), dessen Laufrad (24) drehbeweglich um die Gegenelektrode (18) des Amperemeters befestigt ist, Drehzahlmessmittel (28, 29) aufweist.

7. Vorrichtung nach einem der Ansprüche 1 bis 6, **dadurch gekennzeichnet, dass** das Gehäuse (2) Fenster (30, 34) zum Ein- und Austritt des Wassers aufweist, die sich jeweils neben dem Durchflussmesser (14) bzw. Amperemeter (16) erstrecken.

8. Vorrichtung nach Anspruch 7, **dadurch gekennzeichnet, dass** ein Fenster (30) radial in einer Oberfläche des Schutzmantels (10) neben dem Amperemeter (16) vorgesehen ist.

9. Vorrichtung nach Anspruch 8, **dadurch gekennzeichnet, dass** das Fenster (30) ein Einstellmittel (32) der Öffnung des Fensters aufweist.

10. Vorrichtung nach einem der Ansprüche 7 bis 9, **dadurch gekennzeichnet, dass** Wasser-Eintritts- und -Austrittsfenster (34) axial in der Oberfläche des Gehäuses neben dem Durchflussmesser (14) vorgesehen sind.

11. Vorrichtung nach einem der Ansprüche 3 bis 10, **dadurch gekennzeichnet, dass** sie Reinigungsmittel des Amperemeters (16) integriert, die automatisch mit dem Betrieb des Durchflussmessers (14) kombiniert sind,.

12. Vorrichtung nach Anspruch 11, **dadurch gekennzeichnet, dass** die Reinigungsmittel eine Bürste (38) aufweisen, die dazu ausgelegt ist, das Amperemeter (16) durch die Wirkung des Durchflussmessers (14) abzureiben.

13. Schwimmbecken, das eine Prüfvorrichtung nach einem der Ansprüche 3 bis 12 aufweist, **dadurch gekennzeichnet, dass** die Prüfvorrichtung in einer Kanalisation von Umlaufwasser montiert ist, die einen geschlossenen Kreislauf mit der Wassermasse des Schwimmbeckens bildet.

14. Schwimmbecken nach Anspruch 13, **dadurch gekennzeichnet, dass** die Messvorrichtung in der Kanalisation montiert ist, derart, dass sich die Plattenelektrode der Vorrichtung in der Ebene der Wasserzirkulation erstreckt.

15. Schwimmbecken nach einem der Ansprüche 13 oder 14, **dadurch gekennzeichnet, dass** ein Filter in der Kanalisation für zirkulierendes Wasser der Prüfvorrichtung vorgeschaltet angeordnet ist, wobei die Verschmutzung des Filters durch Durchflusswerte nachgewiesen wird, die durch die Vorrichtung zur Überprüfung des Gehalts an Oxidationsmittel gemessen werden.

## Claims

1. Device for monitoring the oxidizing power of water circulating in a pipeline, comprising flow-rate sensor means (14) and amperometric sensor means (16) for detecting the oxidizing agent ratio in the water, which are connected to one another within a same probe housing (2) fitting laterally through said pipeline wall so that both said sensor means are immersed in the circulating water, **characterised in that** said flow rate sensor means consist of a flowmeter (14) including a turbine, whereas said oxidizing ratio sensor means consist of an ammeter (16) including two electrodes (18, 20), one of which extends along the axis of said housing and bears said turbine of the flowmeter (14), which is mounted to rotate freely about said electrode.

2. Device according to Claim 1, **characterised in that** said housing (2) has a casing of circular cross section.

3. Device according to Claim 1 or 2, **characterised in that** it further includes means for maintaining the turbine in its axial position.

4. Device according to Claim 3, **characterised in that** the position-maintaining means consist of a stop (26) arranged at the bottom of the probe housing (2).

5. Device according to any one of the preceding claims, **characterised in that** the ammeter (16) includes a flat electrode (20) and a counter-electrode (18) projecting from said electrode, said counter-electrode being that one of the two electrodes which bears the flowmeter (14).

6. Device according to Claim 5, **characterised in that** the flowmeter (14) with the turbine (24) mounted to rotate freely about the ammeter counter-electrode (18) includes revolution-counter means (28, 29).

7. Device according to any one of Claims 1 to 6, **characterised in that** said housing (2) includes windows (30, 34) for the input and output of water, which extend respectively opposite the flowmeter (14) and opposite the ammeter (16).

8. Device according to Claim 7, **characterised in that** a window (30) is provided radially in the surface of the casing (10) opposite the ammeter (16).

9. Device according to Claim 8, **characterised in that** said window (30) includes a means (32) for adjustment of the opening of said window.

10. Device according to any of Claims 7 to 9, **characterised in that** windows (34) for input and output of water are provided axially in the surface of the casing, opposite the flowmeter (14).

11. Device according to one of Claims 3 to 10, **characterised in that** it incorporates means for cleaning the ammeter (16) automatically, which are linked to the operation of the flowmeter (14).

12. Device according to Claim 11, **characterised in that** the cleaning means include a brush (38) which is suitable to rub the ammeter (16) by virtue of the action of the flowmeter (14).

13. Swimming pool including a monitoring device according to one of Claims 1 to 12, **characterised in that** the monitoring device is fitted in a water-recirculation pipeline which forms a closed circuit with the mass of water of the swimming pool.

14. Swimming pool according to Claim 13, **characterised in that** the measuring device is fitted in said pipeline so that the flat electrode of the device lies flat with the plane the water circulates in.

15. Swimming pool according to any of Claims 13 or 14, **characterised in that** a filter is arranged in said water-recirculation pipeline upstream of the monitoring device, the clogging of the filter being detected from values of the flow-rate of the water circulating in the pipeline which are measured by the device for monitoring the oxidizing rate.
